# EUROPEAN PATENT APPLICATION

(11) **EP 4 248 891 A1**
(43) Date of publication of application: **27.09.2023**
(21) Application number: 22164172.3
(22) Date of filing: 24.03.2022
(51) Int. Cl.: A61B 18/00, A61B 18/02

(54) **AN ABLATION SYSYTEM FOR PERFORMING ABLATION**

(71) Applicant: Koninklijke Philips N.V., 5656 AG Eindhoven (NL)
(72) Inventor: SCHWARTZ, Yitzhack, Eindhoven (NL); OREN, Eitan, Eindhoven (NL); GLASSNER, Lior Shlomo, Eindhoven (NL)
(74) Representative: Philips Intellectual Property & Standards

(57) **Abstract**

A mechanism for producing an indicator of occlusion for an anatomical cavity by an ablation balloon. The electrical response(s) of one or more first electrodes are processed to generate the indicator. The first electrode(s) are positioned distally to the ablation balloon such that immediately after ablation has been initiated, the ablation has no or negligible effect on the electrical response(s) of the first electrode(s). Each electrical response is responsive to a dielectric property, such as relative permittivity, of material surrounding the first electrode. This dielectric property changes responsive to occlusion and non-occlusion of the anatomical cavity by the ablation balloon.

## Description

### FIELD OF THE INVENTION

The present invention relates to the field of ablation and in particular to ablation systems.

### BACKGROUND OF THE INVENTION

Ablation systems are commonly used to ablate the bounds of an anatomical cavity. An ablation system typically comprises a catheter assembly that carries an ablation balloon. The ablation balloon is moved to a desired occlusion location to occlude the anatomical cavity. The ablation balloon is then controlled or configured to perform ablation against the bounds of the occluded portion of the anatomical cavity.

To achieve appropriate ablation for meeting a clinical need, it is necessary for the ablation balloon to completely occlude the anatomical cavity at the desired occlusion location before and during the ablation procedure. Failure to completely occlude the anatomical cavity would result in incomplete ablation at the relevant location, which would not achieve a desired medical effect.

There is therefore a desire to identify successful occlusion of the anatomical cavity and to improve the monitoring and identification of continued occlusion at the anatomical cavity.

### SUMMARY OF THE INVENTION

The invention is defined by the claims.

According to examples in accordance with an aspect of the invention, there is provided an ablation system for performing ablation on the bounds of an anatomical cavity of a subject. The ablation system comprises a catheter assembly and a processing system.

The catheter assembly comprises: an ablation balloon configured to controllably occlude a portion of the anatomical cavity and be controllable and/or configurable to perform ablation on the bounds of the occluded portion of the anatomical cavity; and one or more first electrodes, located distally to the ablation balloon, wherein each first electrode is configured to obtain a respective electrical response, wherein the electrical response is responsive to a dielectric property of material surrounding the first electrode.

The processing system comprises: an input interface configured to receive an electrical response from each of the one or more first electrodes of the catheter assembly; a processing module configured to process any electrical response of the one or more first electrodes received by the input interface to generate an indicator indicating a predicted measure of a dielectric property of liquid upstream of the ablation balloon; and an optional output interface configured to output the generated indicator.

The one or more first electrodes are spaced apart from the ablation balloon such that, for at least a first time period of at least one second after an ablation procedure is initiated by the ablation balloon, the ablation performed by the ablation balloon has negligible effect on the electrical response obtained by each first electrode.

Embodiments thereby provide a reliable mechanism that facilitates the continued monitoring of an occlusion immediately after ablation has been initiated. By sufficiently spacing the first electrodes away from the ablation balloon, the electrical responses of the first electrodes do not (significantly or perceptibly) react to the ablation during at least a first time period. This allows any leakage past the balloon to be spotted. This is particularly advantageous for providing feedback during the start of the ablation procedure, as the initiation of ablation can cause the ablation balloon to move, e.g., due to changes in the properties of the balloon resulting from the start of ablation.

The proposed mechanism thereby relies upon a recognition that at least one of the catheter electrodes should be positioned or distanced from the ablation balloon, and that such an electrode or electrodes can provide or contribute to a reliable and continued monitoring of the occlusion provided by the ablation balloon.

Approaches provide a system for early-stage and real-time identification of leakages past the ablation balloon at the start of ablation, thereby allowing an operator of the ablation system to react to leakage (e.g., by aborting ablation and/or repositioning the ablation balloon).

Preferably, the ablation balloon is a cryoablation balloon configured to controllably receive a cooling fluid for performing the ablation on the bounds of the occluded portion of the anatomical cavity. Cryoablation provides a "one-shot" approach for ablating the bounds of an anatomical cavity.

Preferably, the spacing between the one or more first electrodes and the ablation balloon is at least 2mm. In more preferred examples, the spacing between the one or more first electrodes and the ablation balloon is at least 6mm.

A spacing of at least 6mm is particularly suited if the ablation balloon is a cryoablation balloon, as it has been experimentally identified that such a spacing will cause (for standard cryoablation procedures and temperatures) no or negligible effect on the electrical response of the first electrode(s). This distance is independent of the size of the balloon.

A smaller distance or spacing (e.g., at least 2mm) is usable for other forms of ablation balloon, e.g., RF ablation balloons.

Preferably, the spacing between the one or more first electrodes and the ablation balloon is no more than 20mm, e.g. no more than 15mm, e.g., no more than 12mm. It has been recognized that positioning the first electrodes more than a certain distance away from the ablation balloon means that effect of occlusion on the dielectric property is significantly attenuated, meaning that it is difficult to distinguish between an occluded and non-occluded state. A distance of no more than 20mm reduces the impact of such attenuation, which is enhanced

Thus, in some examples, the spacing between the one or more first electrodes and the ablation balloon is from 2mm to 20mm, e.g., from 2mm to 15mm, e.g., from 2mm to 12mm, e.g., from 6mm to 20mm, e.g., from 6mm to 15mm, e.g., from 6mm to 12mm.

In some examples, the position of the one or more first electrodes can be changed independently of the position of the ablation balloon. This allows an operator of the ablation system the flexibility or option to position the first electrodes away from the ablation balloon and/or further distance the first electrodes from the ablation balloon.

In some examples, the processing module of the processing system is configured to average the electrical response received by each first electrode to produce the indicator. An average of the electrical responses will reduce the effect of noise.

In some examples, the catheter assembly comprises a plurality of catheter electrodes, each catheter electrode being located distally to the ablation balloon and configured to obtain a respective electrical response, wherein the electrical response is responsive to a dielectric property of material surrounding the catheter electrode.

The plurality of catheter electrodes comprises the one or more first electrodes and the input interface of the processing system is configured to an electrical response from each of the plurality of catheter electrodes of the catheter assembly.

In such examples, the processing module may be configured to: process the electrical response of the plurality of catheter electrodes to identify each electrical response of the one or more first electrodes; and process the identified electrical response of the one or more first electrodes to generate the indicator indicating the predicted measure of the dielectric property of liquid upstream of the ablation balloon.

In this way, the processing module may be able to identify the electrical responses of the first electrode(s) amongst a plurality of electrical responses. The electrical response of other catheter electrodes (i.e., catheter electrodes that are not first electrode) may be excluded or prohibited from being used in the generation of the indicator. This avoids use of electrical responses that may have been affected by ablation, increasing an accuracy of the indicator.

The processing module may configured to process the electrical response of the plurality of catheter electrodes to identify each electrical response of the one or more first electrodes by: receiving, via the input interface, an indication that ablation using the ablation balloon has been initiated; and identifying each electrical response, of the monitored electrical responses, that has not been significantly distorted or affected by the ablation performed by the ablation balloon during the first time period as an electrical response of the one or more first electrodes.

In some examples, the processing module is configured to perform the step of identifying each electrical response, of the monitored electrical responses, that has not been significantly distorted or affected by the ablation performed by the ablation balloon during the first time period by: identifying any electrical responses that are at or near zero as not being an electrical response of the one or more first electrodes.

In another example, a significant distortion can be identified by identifying a sudden change in the value of the monitored electrical response, e.g., a change of more than Y% over a period of Z seconds. Y and Z are predetermined values. Y may be a value greater than or equal to 25, e.g., a value greater than or equal to 50, e.g., a value greater than or equal to 75. Z may be no greater than 0.5, e.g., no greater than 0.3. This approach provides a mechanism for identifying sudden changes or distortion attributable to a result of the effect of ablation (e.g., rather than a result of a shifting or movement of the balloon).

The one or more first electrodes may comprise or be a plurality of first electrodes arranged around a hypothetical circle or spiral. This approach helps improve the identification of any leaks past the ablation balloon, i.e., a change in the occlusion status. In particular, by arranging the first electrodes in a circle or spiral, a leak past a particular side of the ablation balloon are more likely to be reflect in a change in one of the electrical responses of the first electrodes.

In some examples, the dielectric property of liquid is a dielectric property that changes response to the presence or absence of a dielectric medium in the liquid upstream of the ablation balloon. A suitable example is a relative permittivity.

The ablation system may further comprise a dielectric medium injection system configured to inject a dielectric medium upstream of the ablation balloon prior to ablation performed by the ablation balloon. This enhances the effect of the electrical response to represent or identify leakage past the ablation balloon.

In some examples, the catheter assembly comprises: a balloon therapy catheter carrying the ablation balloon; and an electrophysiology catheter carrying the one or more electrodes, wherein the electrophysiology catheter is movable through a lumen of the balloon therapy catheter.

There is also proposed a computer-implemented method for aiding ablation performed on a subject, wherein the ablation is performed using a catheter assembly comprising: an ablation balloon configured to controllably occlude a portion of the anatomical cavity and be controllable and/or configurable to perform ablation on the bounds of the occluded portion of the anatomical cavity; and a plurality of catheter electrodes, located distally to the ablation balloon, wherein each catheter electrode is configured to obtain a respective electrical response, wherein the electrical response is responsive to a dielectric property of material surrounding the first electrode.

The computer-implemented method comprises: receiving, at an input interface, an electrical response from each of the plurality of catheter electrodes of the catheter assembly; processing, using a processing module, the electrical response of the plurality of catheter electrodes to identify each electrical response of one or more first electrodes of the catheter assembly, the one or more first electrodes being spaced apart from the ablation balloon such that, for at least a first time period of at least one second after an ablation procedure is initiated by the ablation balloon, the ablation performed by the ablation balloon has negligible effect on the electrical response obtained by each first electrode; and processing, using the processing module, the identified electrical response of the one or more first electrodes to generate an indicator indicating the predicted measure of the dielectric property of liquid upstream of the ablation balloon.

There is also proposed a computer program product comprising computer program code means which, when executed on a computing device having a processing system, cause the processing system to perform all of the steps of a herein described (computer-implemented) method.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a catheter assembly during an ablation procedure;
Figure 2 illustrates an ablation system;
Figures 3 and 4 illustrate the effect of occlusion on the electrical response of a catheter electrode positioned distally to an ablation balloon;
Figure 5 illustrates a processing system; and
Figure 6 illustrates a method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for producing an indicator of occlusion for an anatomical cavity by an ablation balloon. The electrical response(s) of one or more first electrodes are processed to generate the indicator. The first electrode(s) are positioned distally to the ablation balloon such that immediately after ablation has been initiated, the ablation has no or negligible effect on the electrical response(s) of the first electrode(s). Each electrical response is responsive to a dielectric property, such as relative permittivity, of material surrounding the first electrode. This dielectric property changes responsive to occlusion and non-occlusion of the anatomical cavity by the ablation balloon.

Embodiments are based on the realization that sufficiently distancing electrode(s) from an ablation balloon means that, for at least a first time period after the start of ablation, the ablation does not affect the electrical response of the sufficiently distanced electrode(s). The electrical response(s) of the electrode(s) can therefore be used to monitor occlusion, even after ablation has begun.

Another aspect recognizes that it is possible to distinguish between electrical responses of sufficiently distanced electrodes and insufficiently distanced electrodes. Thus, it is possible to automatically identify which electrical responses of a plurality of catheter electrodes should be used in the generation of the indicator.

Embodiments are particularly advantageous for use during an ablation procedure, and provide a mechanism for providing a live or real-time feedback on an ongoing ablation.

In the context of the present invention, the term "upstream" refers to a direction against the average flow of liquid (e.g., blood). Conversely, the term "downstream" refers to a direction parallel or along the average flow of liquid. In the context of a cardiac system, directional flow is defined by the beating of the heart.

For the sake of improved contextual understanding, an exemplary catheter assembly for use with an ablation system is hereafter described. The operation of the catheter assembly is also elucidated.

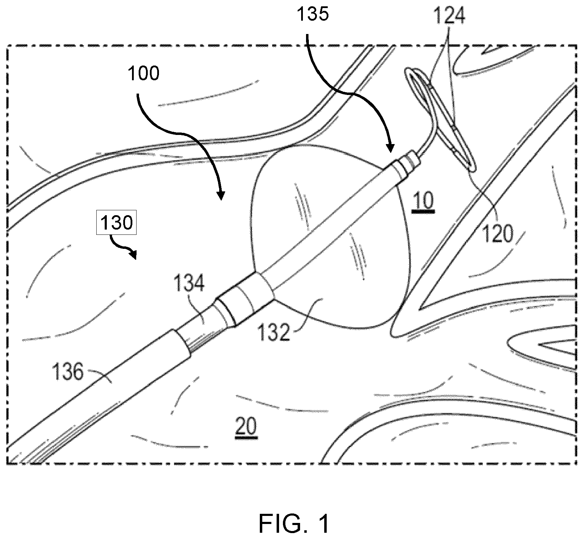

Figure 1 therefore provides an exemplary embodiment of a catheter assembly 100 that may be used in an ablation therapy.

The assembly includes a balloon therapy catheter 130 having an ablation balloon 132 that can be positioned at a desired occlusion location of an anatomical cavity. Here, the desired occlusion location is exemplified as an ostium of a PV 10 within an overall anatomical cavity 20 representing the left atrium (LA) and PVs connected thereto of heart of a subject. The balloon therapy catheter 130 in this case is a balloon ablation catheter. The skilled person would appreciate other use case scenarios and positions for balloon therapy.

Thus, the LA and PV represent an example of features that define an anatomical cavity. Commonly, it is desirable to occlude the anatomical cavity at (the ostium of) the PV and ablate the PV.

The ablation balloon 132 here comprises an inflatable balloon (shown in inflated condition), which is attached to a distal portion of a flexible elongate member 134 of the balloon therapy catheter 130. The elongate member 134 has a member tip 135 at a distal position to the balloon 132.

The ablation balloon 132 is configured to controllably occlude a portion of the anatomical cavity and be controllable and/or configurable to perform ablation on the bounds of the occluded portion of the anatomical cavity.

In this example, the balloon 132 is a cry balloon configured to be inflated and then at least partially filled with a cooling fluid (refrigerant) to cool the cryoballoon to a temperature (e.g., below -65° C) that causes an electrically-isolating lesion or firewall in the tissue when in contact with the boundary or bounds of the occluded portion of the cavity. For example, the cryoballoon 132 may be in fluid communication with a source or reservoir of cooling fluid via one or more fluid lines positioned within the flexible elongate member 134. Further, the cryoballoon 132 may be in fluid communication with an air or gas source, e.g., for balloon inflation, via one or more fluid lines positioned within the flexible elongate member 134.

The elongate flexible member 134 comprises a lumen extending from its proximal end (not shown) to its distal tip 135 and is suitable for slidably receiving an electrophysiology (EP) catheter 120, sometimes also referred to as a mapping catheter. The EP catheter comprises one or more (e.g. a plurality of) separately addressable electrodes 124, which can be labelled catheter electrodes, disposed along a distal tip thereof. The EP catheter is extendible from or retractable into the lumen via the (distal) tip 135 of the flexible member 134. The EP catheter may be removed from the member 134 entirely if needed, e.g., to disassemble the catheter assembly 100.

The catheter electrodes 124 are therefore positioned distally to the ablation balloon when the catheter assembly 100 is formed or assembled.

The elongate member 134 may be movably disposed within a sheath 136. When the balloon is in a deflated state the whole of the member 134 including the deflated balloon may be retracted into and through the sheath 136. Of course, there may be no need to deflate a sufficiently flexible balloon. When protruding out a distal end of a flexible steering sheath 136, the balloon may be inflated as shown.

The flexible steering sheath 136 has pull wires to be operated by a user to steer the sheath and guide the balloon therapy catheter as well as the EP catheter if contained therein.

As previously explained, the EP catheter 120 comprises one or more (a plurality of) mutually electrically separated electrodes 124, or catheter electrodes, disposed along an elongate catheter tip member 126. Only three of those are shown in the Figure 1 for clarity only. In some embodiments, the EP catheter 120 comprises between 8 and 10 electrodes. However, the catheter 120 can include other numbers of electrodes, including 2, 4, 6, 14, 15, 20, 30, 60, or any other suitable number of electrodes. More than 10 electrodes are preferred and more than 15 are even more preferred.

Examples of balloon therapy catheters manufactured by Medtronic^{™} are the Arctic Front^{™} Family of Cardiac Cryoablation Catheters and/or the FlexCath^{™} Advance Steerable Sheath. Examples of EP catheters that work with the aforementioned examples manufactured by the Medtronic^{™} are the Achieve^{™} and Achieve Advance^{™} Mapping Catheters Families. However, other types of such catheters for example from other manufacturers may be used in conjunction with the current mechanism.

In some other embodiments, rather than a cryoballoon, the ablation balloon could be formed of an inflatable balloon that mounts a second set of electrodes, which can be labelled ablation electrodes. Such electrodes are typically used for providing ablation energy in the form of RF power. Further details regarding suitable catheters and assemblies can be found in, for example, U.S. Patent No. 6,002,955, titled "Stabilized Electrophysiology Catheter and Method for Use," the entirety of which is hereby incorporated by reference.

Historically, if the ablation balloon carries a second set of electrodes is present, the EP catheter is not used and/or does not form part of a catheter assembly, as the second set of electrodes can be used to perform the same function, thereby reducing device complexity.

The movement of different catheters and sheaths may be controlled by a user using a catheter control device or handle. The handle is not shown in Figure 1 and such control is known in the art. For example, the EP catheter may be controllably manually or via an automated mechanism to be retractable within and extendable from the lumen of the member 134 of the balloon therapy catheter 130. It may even be entirely removed from the lumen and for example replaced with a guide wire or other wire or catheter. The EP catheter 120 and therewith its electrodes may thus be moved relative to the inflatable balloon or member tip 135 for example. Likewise, the member 134 may be controlled by the handle and moved with respect to the sheath 136.

The EP catheter elongate tip member is configured to be positioned distally of the cryoballoon for example during a therapy procedure, and may be biased, shaped, or otherwise structurally configured to assume a shape, such as a circular one in which the electrodes are spaced within one plane or a spiral one in which the electrodes are spaced from one another about one or more planes. For example, the elongate tip member can be similar to a spiral mapping catheter (SMC) in which electrodes are distributed along an elongate tip member having assumed or configurable to adopt a spiral configuration.

Using the controls described herein above, in some embodiments, a following procedure can be used. First, the sheath 136 having retracted therein the balloon therapy catheter with balloon in deflated state and the EP catheter 120 is first introduced into the LA via known procedures to guide the balloon and EP catheter 120 to the ablation site. Alternatively, a guidewire taking the place of the EP catheter may first be introduced in the PV over which the balloon therapy catheter including the flexible member 134, deflated balloon and sheath 136 are guided towards the LA before the guide wire is replaced with the EP catheter. Once the balloon therapy catheter 130 is in the LA, the flexible member 134 may be extended from the sheath 136 to expose the inflatable balloon 132 in order to inflate it as shown in Figure 1 The EP catheter 120 may now be extended from the member 134 out of the tip 135 to be spiral or lasso shaped and positioned in the PV. Once positioned, the member 134 may be manipulated to bring the inflated balloon towards the PV ostium so that a full circumference of the balloon 132 is in contact with an entire circumference of the PV 10 near or at the ostium. In this way, a full occlusion of the PV at the ostium may be achieved and by cooling of the balloon (or appropriate activation/control of electrodes positioned on the balloon) the ablation can be delivered around the entire circumference with an increased likelihood that the ablation results in complete electrical isolation.

The present invention relates to an approach for detecting any movement of the ablation balloon at the start of an ablation procedure, and in particular, to detect whether full occlusion of the anatomical cavity by the balloon is preserved. Described approaches can further define an amount of occlusion immediately after ablation has begun, thereby aiding a clinician in determining whether or not to continue with the ablation.

The herein proposed approach provides a mechanism for immediate and/or real-time feedback on the predicted occlusion success during a first phase of ablation performed using the ablation balloon.

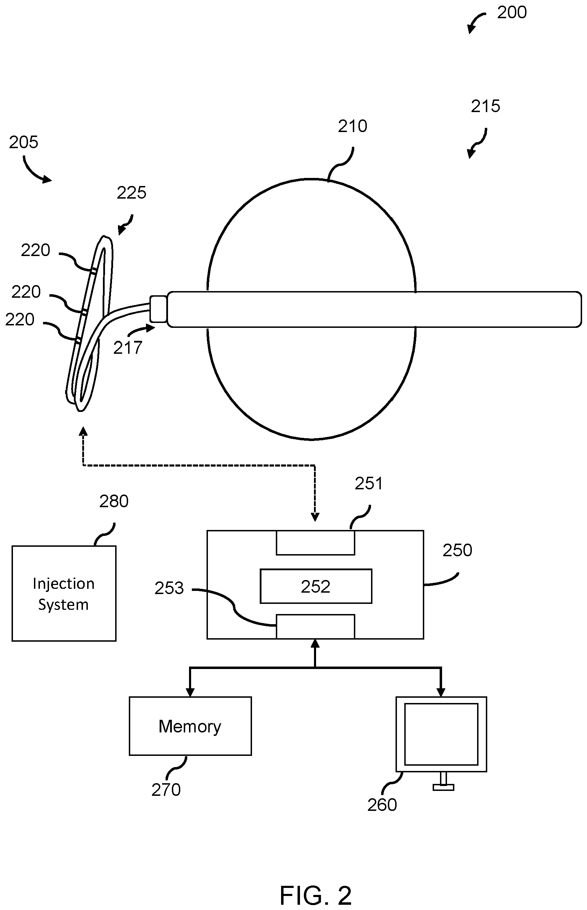

Figure 2 illustrates an ablation system 200 according to an embodiment. The ablation system 200 comprises a catheter assembly 205 and a processing system 250.

The catheter assembly 205 comprises an ablation balloon 210, which can be positioned to occlude an anatomical cavity of a subject, e.g., using an approach previously described. The ablation balloon is also controllable and/or configurable to perform ablation on the bounds of the occluded portion of the anatomical cavity.

For instance, the ablation balloon may be filled with a cooling fluid to perform cryoablation. As another example, the ablation balloon may comprise one or more ablation electrodes positioned around the circumference of a balloon, and the ablation electrodes may be controlled to perform ablation to the bounds of the occluded portion, e.g., using an RF ablation technique such as those previously described.

The catheter assembly 205 further comprises one or more catheter electrodes 220 that are positioned distally to the ablation balloon 210. The skilled person will appreciate that a catheter assembly 205 defines a distal end (being the end that is inserted into the subject) and a proximate end (being the end closest to a clinician). Elements that are positioned distally to another element are positioned closer to the distal end than the other element.

In the illustrated example, the ablation balloon 210 is mounted on or forms part of a balloon therapy catheter 215. Similarly, the one or more catheter electrodes 220 are mounted on or form part of an electrophysiology catheter (EP catheter) 225. The EP catheter is sized to be moveable through a lumen 217 of the balloon therapy catheter 215. This approach allows the catheter electrodes to be repositioned with respect to the ablation balloon.

However, in alternative examples, the ablation balloon 210 and the one or more catheter electrodes 220 are mounted on or form part of a same catheter.

The processing system 250 is configured to receive an electrical response from each catheter electrode 220 at an input interface 251. The electrical response represents electrical activity at the said catheter electrode. In particular, the electrical response may be responsive to changes in a dielectric property, e.g., the relative permittivity, of liquid or matter surrounding the catheter electrode. Changes in the dielectric property or properties causes a change in the electrical response at the catheter electrode.

The processing system 250 also comprises a processing module 252 configured to process the electrical response(s) received by the input interface 251 to generate an indicator indicating a predicted measure of a dielectric property of liquid upstream of the ablation balloon.

The generated indicator may, for instance, be an indicator of relative permittivity of the liquid upstream of the ablation balloon. The generated indicator may be a numeric value that changes responsive to the permittivity of the liquid upstream of the ablation balloon.

The generated indicator indicates whether or not the ablation balloon has occluded the anatomical cavity. If the generated indicator is a numeric value, then it also represents a measure of the occlusion.

In a particular example, the electrical response may be an electrical response to a locally generated electric field, e.g., an electric field generated by one of the catheter electrodes 220 of the catheter assembly 205. The electric field may be generated at a predetermined frequency, and the electrical response to the locally generated electric field at the predetermined frequency can be identified, for example, using one or more frequency discrimination techniques (e.g., a Fourier-based transform such as an FFT) and/or frequency filters. The predetermined frequency may be a frequency in the range of 10kHz to 50kHz, e.g., 20kHz. Greater frequencies would be less responsive to changes in a dielectric property.

An electrical response may, for instance, be a voltage measurement, e.g., a voltage difference between a catheter electrode 220 of the catheter assembly and a reference electrode. The reference electrode may, for instance, be an external electrode positioned on the body of the subject, e.g., the leg of the subject. A voltage measurement such as this has been identified as being particularly responsive to a change in a dielectric property of liquid surrounding the electrode taking the voltage measurement.

More specifically, the/each electrical response (used for generating the indicator) may be a voltage measurement at a predetermined frequency, the predetermined frequency being a frequency of a locally generated electric field, wherein the locally generated electric field is generated by one of the catheter electrodes.

As previously explained, the indicator of the predicted measure of a dielectric property represents or indicates an occlusion of the anatomical cavity by the ablation balloon.

It has been recognized that occluding an anatomical cavity affects the dielectric properties of liquid upstream from the occluded portion of the anatomical cavity. Thus, there is an effect on the electrical response of a catheter electrode when the ablation balloon occluded the anatomical cavity.

Experimental evidence has identified that, at a catheter electrode positioned distally to the ablation balloon, there is about a 7% change in a voltage response to a locally generated electric field when the ablation balloon moves from a non-occluding position to an occluding position. Other forms of responses (e.g., impedance or the like) change to a similar extent.

The effect of the change in the dielectric property of the liquid can be enhanced by the introduction of a dielectric medium to the anatomical cavity upstream of the balloon catheter. A dielectric medium is a medium that has a different value for the (target) dielectric property than blood. One suitable example of a dielectric medium is saline. Another suitable example is a contrast agent or enhancing agent, sometimes called a dye. Other clinically safe dielectric mediums will be apparent to the skilled person.

If the ablation balloon occludes the anatomical cavity, then a dielectric medium injected upstream of the occlusion will pool at the distal end of the ablation balloon. The electrical response of the catheter electrode(s) will therefore reflect the presence of the dielectric medium, which affects the dielectric property of the liquid in the vicinity of the electrode(s).

If the ablation balloon does not (fully) occlude the anatomical cavity, then the dielectric medium will be able to flow past the ablation balloon. Thus, the dielectric property of the liquid in the vicinity of the electrode(s) will match an expected value for blood.

Experimental evidence has identified that, at a catheter electrode positioned distally to the ablation balloon, there is about a 30% change in a voltage response to a locally generated electric field when the dielectric medium pools at the distal end of the ablation balloon (i.e. there is occlusion) compared to when the dielectric medium is allowed to flow past the ablation balloon (i.e., there is no occlusion).

It has been recognized that ablation in the vicinity of a catheter electrode will affect the electrical response of the catheter electrode, particularly the electrical response to a dielectric property of a liquid upstream or distal to the ablation balloon. This is due to sudden changes in temperature (e.g., for a cryoablation procedure) and/or the introduction of noise (e.g., for an RF ablation procedure). This has historically meant that it was not considered possible to monitor occlusion once ablation had begun.

However, it herein recognized that sufficiently distancing a catheter electrode from the ablation balloon means that, for at least an initial/first period of time after ablation begins, the ablation has minimal or negligible direct effect on the electrical response of the catheter electrode.

The precise distance required is dependent upon the type of ablation performed by the ablation balloon. For instance, for a cryoablation procedure, a distance of at least 6mm, and preferably at least 10mm, from the ablation balloon is recommended to ensure that the ablation performed by the ablation balloon has negligible effect on the electrical response obtained by a catheter electrode. As another example, for an RF ablation procedure, a distance of at least 2mm, and preferably at least 5mm, from the ablation balloon is recommended to ensure that the ablation performed by the ablation balloon has negligible effect on the electrical response obtained by a catheter electrode.

To exploit this recognition, when ablation is initiated (via the ablation balloon), the catheter assembly 205 is configured such that the catheter electrodes 220 are positioned such that one or more of the catheter electrodes, which can be labelled "first electrodes", are separated or spaced apart from the ablation balloon 210. The separation or spacing is such that the ablation performed by the ablation balloon has negligible effect on the electrical response obtained by each first electrode for at least a first time period.

The first time period is at least 1 second, e.g., at least 3 seconds, e.g., at least 6 seconds.

In this way, occlusion of the anatomical cavity can continue to be reliably monitored after the onset of ablation for at least the first period of time, e.g., to check whether the ablation balloon has moved at the start of ablation (e.g., as a result of the ablation initiation process, such as filling the balloon with cooling fluid for a cryoablation process).

The processing system 250 may be configured to identify the electrical responses of the first electrodes. In other words, the processing system may be configured to discriminate between electrical responses of the first electrodes and the electrical responses of second electrodes, being electrodes of the catheter electrodes 220 that are too close to the ablation balloon such that the ablation has a marked effect on the electrical response.

In this way, the processing system 250 may be able to identify the electrical response(s) of the first electrode(s).

The processing module 252 of the processing system 250 may therefore be configured to process the electrical response of the plurality of catheter electrodes to identify each electrical response of the one or more first electrodes; and process each identified electrical response of the one or more first electrodes to generate the indicator indicating the predicted measure of the dielectric property of liquid upstream of the ablation balloon.

The indicator of the predicted measure of the dielectric property thereby reflects an occlusion state of the anatomical cavity, i.e., an extent to which the anatomical cavity is occluded by the ablation balloon.

The electrical response, or a filtered version thereof, itself may represent a suitable indicator of the dielectric property. It has previously been explained how an electrical response can reflect or represent the dielectric property.

In one example, the processing module is configured to process the electrical response of the plurality of catheter electrodes to identify each electrical response of the one or more first electrodes by receiving, via the input interface, an ablation indication that ablation using the ablation balloon has been initiated.

Identifying each electrical response of the one or more first electrodes effectively comprises determining which of the electrical responses received at the input interface are electrical responses of first electrodes, being electrodes that are separated or spaced apart from the ablation balloon such that the ablation performed by the ablation balloon has negligible effect on the electrical response obtained by said electrode for at least a first time period.

A separate ablating system (not shown) may be configured to control the ablation performed by the ablation balloon. The ablating system may be configured to provide the ablation indication (e.g., an ablation initiation signal) to the processing system.

The processing module is then configured to identify each electrical response, of the monitored electrical responses, that has not been significantly distorted or affected by the ablation performed by the ablation balloon during the first time period as an electrical response of the one or more first electrodes.

Thus, the processing module identifies any electrical response that have not been distorted by the ablation as being an electrical response of a first electrode.

In one example, the processing module is configured to perform the step of identifying each electrical response, of the monitored electrical responses, that has not been significantly distorted or affected by the ablation performed by the ablation balloon during the first time period by identifying any electrical responses that are at or near zero as not being an electrical response of the one or more first electrodes.

It has been recognized that a zero or near-zero electrical response is indicative that the corresponding electrode (producing the electrical response) has been distorted or affected by an ablation procedure. For instance, a cryoablation procedure will cause electrodes that are too proximate to the ablation balloon to freeze, which will effectively cause the electrical response of such electrodes to drop to zero.

As another example, it has been recognized that a sudden change in the value of the monitored electrical response is indicative of the electrical response (and therefore catheter electrode) being significantly distorted or affected by the ablation performed by the ablation balloon.

Identifying electrical responses that have undergone a sudden change may comprise identifying any electrical responses that have had a change of more than Y% over a period of Z seconds. Y and Z are predetermined values. Y may be a value greater than or equal to 25, e.g., a value greater than or equal to 50, e.g., a value greater than or equal to 75. Z may be no greater than 0.5, e.g., no greater than 0.3. This approach provides a mechanism for identifying sudden changes or distortion attributable to a result of the effect of ablation (e.g., rather than a result of a shifting or movement of the balloon).

Any electrical response that has not been identified as undergoing a sudden change may be defined or identified as being an electrical response of a first electrode.

During the first time period, i.e., immediately after ablation, the processing module 252 is configured to process the electrical response(s) provided by any such first electrodes to produce the indicator. Thus, the electrical response(s) (of any of the catheter electrode(s) used to generate the indicator during the first time period are limited or restricted to only the electrical response(s) of the first electrode(s). Any electrical response(s) from other catheter electrodes (if present) received at the input interface is not used or is excluded from use in the generation of the indicator.

The first time period is at least 1 second, e.g., at least 3 seconds, e.g., at least 6 seconds. The first time period begins at the initiation of ablation or receipt of an indicator of initiation of ablation.

Thus, the processing module may be configured to receive, via the input interface, an indication that ablation has been initiated. Responsive to this indication, the processing module 252 may process the electrical response(s) to produce the indicator, wherein the electrical response(s) (of any of the catheter electrode(s)) used during the first time period are limited or restricted to only the electrical response(s) of the first electrode(s). Any electrical response(s) from other catheter electrodes (if present) received at the input interface is not used or is excluded from use in the generation of the indicator.

The processing module 252 may be configured to average the electrical response(s) provided by each first electrode to produce the indicator. This provides a quick and easy reference for establishing whether or not the ablation balloon occludes the anatomical cavity.

Another approach for producing an indicator is to select one of the electrical responses (associated with one of the first electrode(s)) to act as the indicator.

As an example, the electrical response that undergoes the biggest change (e.g., percentage change) may be selected to act as the indicator. This is particularly advantageous as different first electrodes will be at different locations. The effect of a small leak past a portion of the ablation balloon is likely to affect the first electrode(s) closer to said portion than others. By selecting the electrical response that undergoes the biggest change as the indicator, identification of a leak and therefore change in occlusion can be more precisely identified.

In other examples, the processing module 252 is configured to generate a plurality of indicators.

Each electrical response may on its own form a separate indicator. Thus, producing an indicator may comprise producing an indicator for each of the first electrodes or a subset of the first electrodes.

As previously explained, the effect of a small leak past a portion of the ablation balloon is likely to affect the first electrode(s) closer to said portion than others. By providing a plurality of indications for each first electrode, it is possible to identify which first electrode is closest to a potential leak and to assess the magnitude and/or effect of the leak (e.g., whether the leak is affecting the electrical response(s) of other first electrodes, which would indicate a size of the leak).

Another approach for producing a plurality of indicators may be performed by, for each of a plurality of sets of first electrodes, determining an average of the electrical responses provided by the set of first electrodes. Each set is different. Each set may comprise a grouping of two or more adjacent catheter electrodes (e.g., catheter electrodes that are positionally located the most proximate to one another compared to other catheter electrodes).

If ablation is not being performed, the processing module 252 may process the electrical response(s) to produce the indicator, wherein the electrical response(s) (of any of the catheter electrode(s) used during the first time period are not limited or restricted to only the electrical response(s) of the first electrode(s). Thus, the electrical response(s) from other catheter electrodes (if present) received at the input interface are permitted for use or are allowed to be used in the generation of the indicator. This can be used to generate the indicator before/after an ablation process.

The skilled person would be readily capable of adapting any above described approach (e.g., averaging or selecting an electrical responses) to produce the indicator from electrical responses of the plurality of electrodes, including more than just the first electrodes.

Thus, the processing module 252 may operate in two modes. In a non-ablation mode, the indicator may be generated using the electrical response(s) from all catheter electrodes. In an ablation mode, which occurs for at least the first time period after ablation is initiated, the indicator may be generating using only the electrical responses from first electrodes, i.e., the electrical responses of any other catheter electrode are not used in generating the indicator.

The processing module may switch between the ablation mode and the non-ablation mode response to an indicator that ablation is (currently) ongoing.

However, the skilled person would appreciate that the non-ablation mode is optional. Rather, a processing module may be configured to continually operate in the described ablation mode.

Of course, further processing may be performed on any electrical response and/or averaged electrical response to generate the indicator.

For instance, a value of an electrical response (or an average value of electrical responses) may be compared to a predetermined threshold, in which not breaching the predetermined threshold indicates that the ablation balloon is not occluding the anatomical cavity and breaching the predetermined threshold indicates that the ablation balloon occludes the anatomical cavity. The indicator may be responsive to the value breaching or not breaching the threshold, e.g., to provide a binary indicator of occlusion.

As another example, a value of an electrical response (or an average value of electrical responses) may be compared to a plurality of predetermined ranges. Each range may define a different category of occlusion (e.g., "Very Good, "Good", "Poor", "Very Poor" or "None"). The predetermined range into which the value of an electrical response or an average value of electrical responses falls may define the value of the predicted indicator.

In this way, each generated indicator may be numeric (e.g., a value of an electrical response or an average value of electrical responses), binary (e.g., a determination of whether or not a value of an electrical response or an average value of electrical responses breaches a predetermined threshold) and/or categorical (e.g., a determination of into which of a plurality of different predetermined ranges a value of an electrical response or an average value of electrical responses falls).

The processing system 250 is preferably configured to operate in real time, e.g., obtain the most recently generated or available value of the electrical response(s) of the first electrode(s) and use the most recently available value(s) to produce the indicator.

The processing system 250 may be configured to repeatedly or continually update the indicator, e.g., using the most recently available value of the electrical response(s) of the first electrode(s). This can advantageously provide a "real-time" feedback on the occlusion state of the ablation balloon.

The processing system 250 may comprise an output interface 253 configured to output the generated indicator.

In some examples, the ablation system 200 comprises a user interface 260 configured to provide a visual representation of the generated indicator. If a plurality of indicators are generated, the user interface 260 may provide a visual representation of each indicator. The operation of the user interface 260 may be controlled by the processing module 252 via the output interface 253.

Providing a visual representation of the generated indicator can supply immediate feedback to a clinician performing an ablation. In particular, the visual representation is able to provide the clinician with information about the occlusion, and whether there is a leakage past the occlusion - i.e., an amount of occlusion.

Thus, the processing system 250 may be configured to control a user interface to provide a visual representation of the indicator(s), e.g., via the output interface 253.

In some examples, the processing module of the processing system is configured to determine, for each first electrode, an indicator indicating a predicted measure of a dielectric property of liquid upstream of the ablation balloon using the electrical response of the first electrode.

The processing module of the processing system may be further configured to control a user interface to provide a visual representation of the determined indicator for each first electrode.

In some examples, the ablation system 200 comprises a storage or memory unit 270 configured to store the generated indicator. The storing of the generated indicator may be controlled by the processing module 252 via the output interface 253.

To benefit from the advantages of improved distinguishability between an occlusion and non-occlusion state in the electrical response of the catheter electrode(s), the ablation system may be configured such that a dielectric medium is injected (immediately) prior to ablation. This allows for improved tracking and monitoring of any leaks at the start of the ablation procedure.

Thus, the ablation system 200 may comprise a dielectric medium injection system 280 configured to inject a dielectric medium upstream of the ablation balloon prior to ablation performed by the ablation balloon.

Suitable examples of dielectric mediums include saline and/or a contrast agent. Alternative names for a contrast agent include an enhancing agent or a dye. Other suitable dielectric mediums will be apparent to the skilled person.

The electrical response(s) received by the input interface of the processing system may be received directly from the catheter assembly, and the processing performed by the processing system may be performed in real-time, e.g., to provide an immediate feedback on the occlusion to a clinician. This is particularly advantageous to aid or guide in the performance of an ablation procedure.

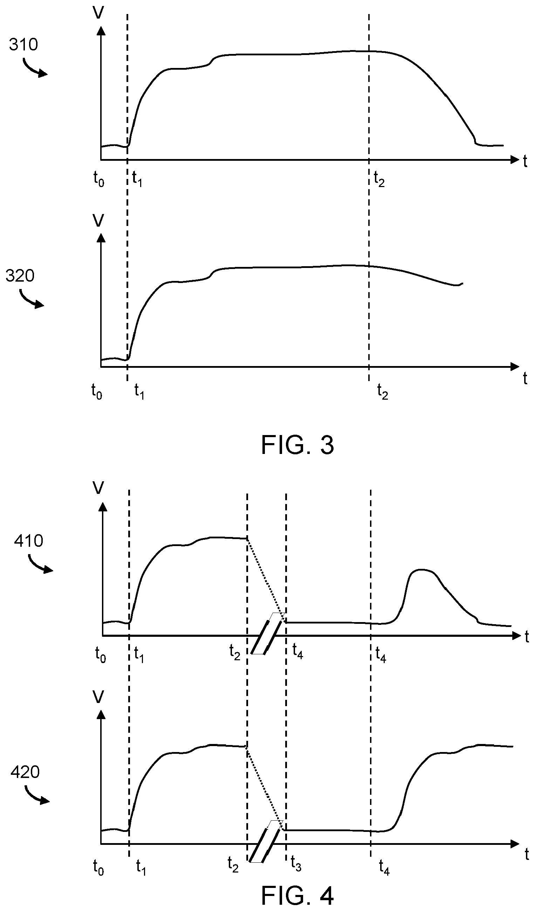

Figures 3 and 4 illustrate the effect of occlusion on the electrical response of a catheter electrode positioned distally to the ablation balloon. The electrical response may represent an indicator of a predicted measure of a dielectric property of liquid upstream of the ablation balloon.

For all waveforms, the electrical response is here a voltage response (V) of the electrode to an electric field generated by a catheter electrode, and is plotted against time t. The voltage response changes responsive to a dielectric property of liquid surrounding the electrode, particularly a relative permittivity of the liquid.

However, other forms of electrical response of an electrode will change in a similar manner (e.g., a capacitive response, an impedance response and so on). These forms of electrical response may be monitored instead of a voltage response.

A first waveform 310 illustrates a first scenario in which occlusion is made at a time t₀, and then a dielectric medium is injected upstream of the occlusion at time t₁. The dielectric medium causes the relative permittivity of the liquid surrounding the catheter electrode to change, causing an increase in the voltage response at the catheter electrode. This indicates that the occlusion was successful.

At a time t₂, ablation is begun. In this scenario, the ablation causes the ablation balloon to stop occluding the anatomical cavity. Thus, there is a sudden drop in the voltage response as the dielectric medium is allowed to flow past the non-occluding ablation balloon.

The voltage response can be monitored in real-time to provide useful information for a clinician performing ablation.

A second waveform 320 illustrates a second scenario. The second scenario differs from the first scenario in that at the ablation at time t₂ does not cause the ablation balloon to stop occluding the anatomical cavity. Thus, there is no sudden drop in the voltage response as the dielectric medium pools at the distal end of the occluding balloon.

A third waveform 410 illustrates a third scenario in which occlusion is made at a time t₀, and then a dielectric medium is injected upstream of the occlusion at time t₁. The dielectric medium causes the relative permittivity of the liquid surrounding the catheter electrode to change, causing an increase in the voltage response at the catheter electrode. This indicates that the occlusion was successful.

There is a span of time between time t₂ and time t₃. This causes the voltage response to drop due to small (but acceptable) leakages across the balloon and/or dilution of the dielectric medium.

At a time t₄, a dielectric medium is injected upstream of the occlusion and an ablation is immediately initiated or begun. In this third scenario, the ablation causes the ablation balloon to stop occluding the anatomical cavity. The dielectric medium causes the voltage response to initially increase (as it reaches the electrode), before decreasing as it makes its way past the non-occluding balloon.

A fourth waveform 420 illustrates a fourth scenario. The fourth scenario differs from the third scenario in that at the ablation at time t₄ does not cause the ablation balloon to stop occluding the anatomical cavity. Thus, there is no sudden drop in the voltage response as the dielectric medium pools at the distal end of the occluding balloon.

It will be clear that the voltage response of a catheter electrode positioned distally to the ablation can be monitored in real-time to provide useful information for a clinician performing ablation.

From the foregoing, it is clear that the electrical response can also act or represent the indicator indicating a predicted measure of a dielectric property of liquid upstream of the ablation balloon.

Thus, in the event that the ablation has caused a change in geometry resulting in even a small leak, an operator viewing the electrical response will be able to immediately detect it and abort the ablation. This can prevent or reduce a length of time required to perform effective ablation of the bounds of the anatomical cavity.

The catheter electrodes could also be used for other tasks or for other functionalities.

For instance, an electrical response of a catheter electrode could be monitored to track the location of the catheter electrode and/or the catheter assembly. Generally, this is performed by monitoring the electrical response of a catheter electrode to externally (to the subject) generated electric fields, particularly crossing electric fields.

The crossing electric fields may be controlled to have a different frequency with respect to one another, which effectively facilitates triangulation of the electrode within an electric field space.

The electric fields to operate in the frequency range of 10-1000 kHz, e.g. 10-500 kHz, e.g. 10-300kHz, e.g. 30-500kHz. These frequency ranges are particularly useful for ensuring penetration of a subject, whilst providing frequencies that attenuate in human tissue without significant damage/injury to the tissue.

The electrical response of a catheter electrode to the electric fields changes as the relative position of the catheter electrode moves about the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the relative position of the catheter electrode(s) and the catheter using a mapping system that monitors a response of the catheter electrode(s) to the crossing electric fields, e.g. by mapping the (electrical) response of the catheter electrode to a relative position within the subject or using a suitable mapping/transfer function, e.g. the "V2R function", to determine the relative position(s) of the electrodes, and therefore the catheter, in a defined space. This defined space is sometimes called an R-space.

Approaches for generating such a mapping function are well established in the art. Example processes are disclosed by the European Patent Applications having publication numbers EP 0775466 A2, EP 3568068 A1 and EP 3607879 A1.

Further explanations and approaches for determining or monitoring a position of a catheter electrode using an electrical response of the catheter electrode are disclosed by the European Patent Applications having publication numbers EP 0775466 A2, EP 3568068 A1 and EP 3607879 A1. Any of these approaches, or others established in the art, could be employed.

Thus, the spatial whereabouts of the catheter electrode and/or the catheter assembly can be determined in three-dimensions.

Information on the determined location of the catheter electrode(s) and/or catheter assembly may be provided to the operator (i.e., performing the ablation) via a visual representation provided at a user interface.

Providing the determined location of the catheter electrode(s) is particularly advantageous if an indicator is generated for each of the first electrodes, wherein the indicator represents a predicted occlusion of the anatomical cavity by the ablation balloon.

In particular, the indicator for each first electrode will indicate a leakage at or past the position of the first electrode. If the position of each first electrode is known, and a leakage is detected at a particular one of the first electrodes, it is possible to manipulate the balloon to stem or block a leakage at or past the particular first electrode.

In this way, an operator is able to attempt repositioning the ablation balloon to reduce or prevent leakage, i.e., to respond to a leak at a particular position or location.

There is therefore proposed an embodiment in which the processing module of the processing system is further configured to process, for each first electrode, the electrical response of the first electrode to determine or predict a location of the first electrode.

The processing module of the processing system may be further configured to control a user interface to provide a visual representation of the location of each first electrode.

Other uses for the determined location(s) of the catheter electrode(s) of the catheter assembly are hereafter described.

In one example, the determined location(s) is/are used to detect which electrodes of a plurality of catheter electrodes are the first electrodes. In particular, the determined location of each catheter electrode may be used to determine whether the electrical response of the catheter electrode would be affected by ablation performed by the ablation balloon, (e.g., by determining a distance between the catheter electrode and the ablation balloon and/or a location at which the ablation balloon is to ablate).

Thus, the processing module may be configured to obtain a location of each of a plurality of catheter electrodes and determine which of the plurality of catheter electrodes is/are (a) first electrode(s) responsive to the determined locations.

In one example, the determined location(s) is/are used in the generation of the indicator that indicates predicted measure of a dielectric property of liquid upstream of the ablation balloon. For instance, the determined location(s) may be used to select one or more of the electrical responses of the first electrodes to use in determining the indicator.

As one example, the electrical response of the first electrode determined to be the most distant from the ablation balloon may be selected for use as the indicator. As another example, the average of the electrical responses of the X most distant first electrodes from the ablation may be determined for use as the indicator, where X is an integer greater than 1 (e.g., 2, 3, 5 or 8).

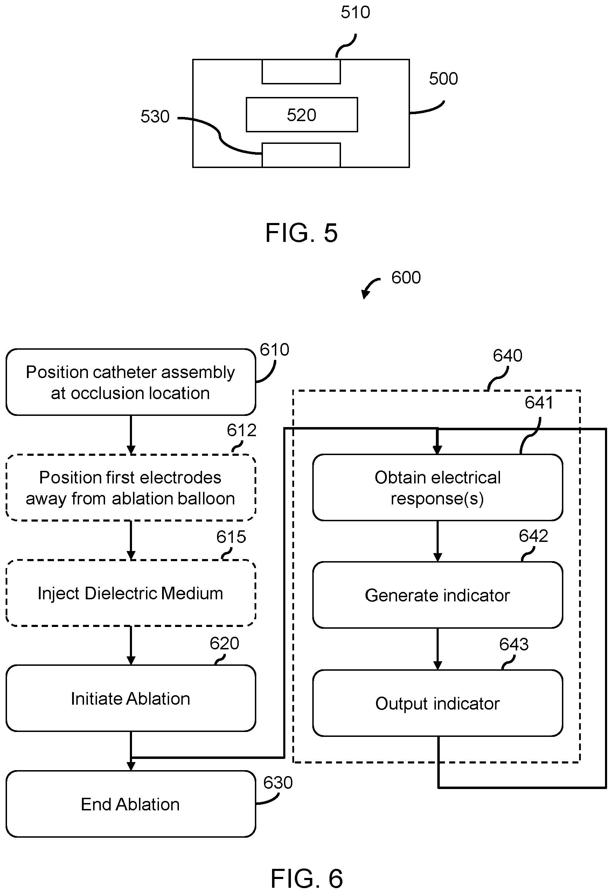

Figure 5 illustrates a processing system 500 for use in an embodiment.

The processing system comprises an input interface 510 configured to receive an electrical response from each of one or more first electrodes of a catheter assembly.

As previously explained, the one or more first electrodes are spaced apart from an ablation balloon of the catheter assembly such that, for at least a first time period of at least one second after an ablation procedure is initiated by the ablation balloon, the ablation performed by the ablation balloon has negligible effect on the electrical response obtained by each first electrode.

The input interface 510 may, for instance, receive an electrical response from a plurality of catheter electrodes of the catheter assembly. The plurality of catheter electrodes includes at least the one or more first electrodes.

The processing system 500 also comprises a processing module 520 configured to process any electrical response (of the first electrode(s)) received by the input interface to generate an indicator indicating a predicted measure of a dielectric property of liquid upstream of the ablation balloon.

If the input interface receives an electrical response from a plurality of electrodes, the processing module 520 may be configured to process the electrical responses to identify the electrical response(s) of the one or more first electrodes. In this way, the processing module 520 may be configured to discriminate or distinguish electrical responses of first electrodes from electrical responses of other electrodes.

Put another way, the processing module 520 may be configured to process the electrical response of the plurality of catheter electrodes to identify each electrical response of the one or more first electrodes; and process the identified electrical response of the one or more first electrodes to generate the indicator indicating the predicted measure of the dielectric property of liquid upstream of the ablation balloon.

In particular, the processing module 520 may be configured to process the electrical response of the plurality of catheter electrodes to identify each electrical response of the one or more first electrodes by: receiving, via the input interface, an indication that ablation using the ablation balloon has been initiated; and identifying each electrical response, of the monitored electrical responses, that has not been significantly distorted or affected by the ablation performed by the ablation balloon during the first time period as an electrical response of the one or more first electrodes.

The indication that ablation has been initiated may be provided by an ablation system (not shown) and/or a user input at a user interface.

In one working example, the processing module 520 is configured to perform the step of identifying each electrical response, of the monitored electrical responses, that has not been significantly distorted or affected by the ablation performed by the ablation balloon during the first time period by identifying any electrical responses that are at or near zero as not being an electrical response of the one or more first electrodes.

This approach recognizes that the effect of ablation, particularly cryoablation, can significantly affect the electrical response of the catheter electrodes that are too close to the ablation balloon of the catheter assembly. In particular, the effect of the ablation may be to cause such catheter electrodes to register no electrical response, e.g., due to a freezing effect.

As another example, it has been recognized that a sudden change in the value of the monitored electrical response is indicative of the electrical response (and therefore catheter electrode) being significantly distorted or affected by the ablation performed by the ablation balloon.

Identifying electrical responses that have undergone a sudden change may comprise identifying any electrical responses that have had a change of more than Y% over a period of Z seconds. Y and Z are predetermined values. Y may be a value greater than or equal to 25, e.g., a value greater than or equal to 50, e.g., a value greater than or equal to 75. Z may be no greater than 0.5, e.g., no greater than 0.3. This approach provides a mechanism for identifying sudden changes or distortion attributable to a result of the effect of ablation (e.g., rather than a result of a shifting or movement of the balloon).

The processing system 500 may further comprise an output interface 530. The output interface may output the indicator indicating a predicted measure of a dielectric property of liquid upstream of the ablation balloon. For instance, the output interface 530 may provide a control signal for controlling the operation of a user interface responsive to the predicted measure of the dielectric property. As another example, the indicator may be provided to a memory or storage unit for storage. As another example, the indicator may be provided to a further processing system for further processing, e.g., performing diagnostic or analysis techniques.

The processing system 500, particularly such a processing system that is able to identify the electrical responses of first electrodes from the electrical responses of a plurality of catheter electrodes, represents another aspect of the inventive concept.

Figure 6 illustrates a method 600 for performing ablation on the bounds of an anatomical cavity of a subject.

The method 600 comprises a step 610 of inserting and moving a catheter assembly until an ablation balloon of the catheter assembly is positioned at a desired occlusion location. The desired occlusion location is a location within an anatomical cavity, the bounds of which are to undergo ablation (e.g., at the PV of the left atrium).

The performance of step 610 can be aided or guided using a position or location tracking system. In particular, the position or location tracking system may track a location of the catheter assembly and/or any catheter electrodes of the catheter assembly, and provide a visual representation of the same, in order to guide the clinician in moving the catheter assembly to the desired occlusion location.

The method 600 then moves to a step 620 of initiating ablation. Thus, ablation is initiated at the desired occlusion location so that the bounds of the anatomical cavity at the occlusion location are ablated.

The steps performed in initiating ablation will vary dependent upon the type of catheter assembly, and particularly the type of ablation balloon. For instance, if the ablation balloon is a cryoablation balloon, then initiating ablation may include a process of providing cooling fluid (e.g., liquid nitrogen) to the ablation balloon to begin cryoablation. For an RF ablation balloon, initiating ablation may include providing electrical signals to electrodes on the ablation balloon for controlling the ablation performed by the RF ablation balloon.

From the perspective of the operator of the catheter assembly, initiating ablation may simply comprise providing a user input indicating a desire to initiate ablation. The process for performing ablation responsive to this user input may be automatic.

The method performs ablation for a period of time, before ending ablation in step 630. The length of the ablation may be predetermined or may be controlled by the operator of the catheter assembly, e.g., for achieving a desired clinical goal.

The method 600 is configured to, in a process 640, determine or produce an indicator indicating a predicted measure of a dielectric property of liquid upstream of the ablation balloon during at least a first time period. This indicator may be provided to the operator of the catheter assembly, e.g., in the form of a visual representation at a user interface.

The first time period is no less than 1 second, e.g., no less than 3 seconds, e.g., no less than 5 seconds, e.g., no less than 6 seconds.

Process 640 may be performed by receiving 641, at an input interface, an electrical response from each of one or more first electrodes of the catheter assembly.

The one or more first electrodes are spaced apart from the ablation balloon such that, for at least the first time period of at least one second after an ablation procedure is initiated by the ablation balloon, the ablation performed by the ablation balloon has negligible effect on the electrical response obtained by each first electrode.

The process 640 may then perform a step 642 of processing, using a processing module, any received electrical response of the one or more first electrodes to generate an indicator indicating a predicted measure of a dielectric property of liquid upstream of the ablation balloon.

Approaches for performing steps 641 and 642 have been previously described.

In particular, step 641 may comprise receiving an electrical response from a plurality of catheter electrodes of the catheter assembly. The plurality of catheter electrodes includes at least the one or more first electrodes.

Step 642 may then comprise using the processing module to process the electrical response of the plurality of catheter electrodes to identify each electrical response of the one or more first electrodes; and then process the identified electrical response of the one or more first electrodes to generate the indicator indicating the predicted measure of the dielectric property of liquid upstream of the ablation balloon.

Processing the electrical response of the plurality of catheter electrodes to identify each electrical response of the one or more first electrodes may be performed by: receiving, via the input interface, an indication that ablation using the ablation balloon has been initiated; and identifying each electrical response, of the monitored electrical responses, that has not been significantly distorted or affected by the ablation performed by the ablation balloon during the first time period as an electrical response of the one or more first electrodes.

The indication may be provided by performing step 620.

In one working example, identifying each electrical response, of the monitored electrical responses, that has not been significantly distorted or affected by the ablation performed by the ablation balloon during the first time period is performed by identifying any electrical responses that are at or near zero as not being an electrical response of the one or more first electrodes.

The process 640 may comprise a step 643 of outputting, at an output interface, the generated indicator. The generated indicator may be provided to the operator of the catheter assembly, e.g., in the form of a visual representation at a user interface. Thus, step 643 may comprise controlling a user interface to provide the visual representation at the user interface.

It will be appreciated that the process 640 is itself an embodiment of the invention. Correspondingly, one embodiment of the invention is a processing system configured to carry out process 640.

The process 640 may be repeated throughout at least the first time period.

In some examples, a version of process 640 is performed before ablation is initiated and/or after ablation is complete. In these circumstances, it is not essential to limit the electrical responses used to generate the indicator using only the electrical responses of the first electrodes.

The method 600 may further comprise a step 615 of injecting a dielectric medium upstream of the occluded portion of the anatomical cavity. Step 615 is performed between steps 610 and 620.

Preferably, step 615 is performed no less than 30 seconds before step 620, more preferably no less than 15 seconds before step 620, more preferably no less than 5 seconds before step 620. This approach recognizes that there is a particular advantage to being able to identify occlusion during the early stages of ablation (e.g., as initiating ablation may cause the ablation balloon to shift). Injecting a dielectric medium shortly before ablation begins improves the distinction or difference between occlusion and non-occlusion in the electrical response of the first electrode(s), thereby improving the indicator produced by process 640.

The method 600 may comprise a step 612 of positioning one or more first electrodes away from the ablation balloon. This can be performed by tracking the location of the first electrodes, e.g., as previously described. The first electrodes may be positioned a minimum distance from the occlusion location, such that ablation performed by the ablation balloon (at the ablation location) has no or negligible effect on the electrical response of the first electrodes.

The precise distance required is dependent upon the type of ablation performed by the ablation balloon. For instance, for a cryoablation procedure, a distance of at least 6mm, and preferably at least 10mm, from the ablation balloon is recommended to ensure that the ablation performed by the ablation balloon has negligible effect on the electrical response obtained by a catheter electrode. As another example, for an RF ablation procedure, a distance of at least 2mm, and preferably at least 5mm, from the ablation balloon is recommended to ensure that the ablation performed by the ablation balloon has negligible effect on the electrical response obtained by a catheter electrode.

Preferably, the spacing between the one or more first electrodes and the ablation balloon is no more than 20mm, e.g. no more than 15mm, e.g., no more than 12mm. It has been recognized that positioning the first electrodes more than a certain distance away from the ablation balloon means that effect of occlusion on the dielectric property is significantly attenuated, meaning that it is difficult to distinguish between an occluded and non-occluded state. A distance of no more than 20mm reduces the impact of such attenuation, which is enhanced

Thus, in some examples, the spacing between the one or more first electrodes and the ablation balloon is from: 2mm to 20mm; from 2mm to 15mm; from 2mm to 12mm; from 5mm to 20mm; from 5mm to 15mm; from 5mm to 12mm; from 6mm to 20mm; from 6mm to 15mm; from 6mm to 12mm; from 10mm to 20mm; from 10mm to 15mm; or from 10mm to 12mm.

Step 612 is performed between steps 610 and 620. Preferably, if both steps 612 and 615 are performed, step 612 is performed before step 615.

Steps 610, 612, 615, 620 and 630 may be performed by a clinician. Process 640 may be performed by a processing system, as previously described.

The skilled person would be readily capable of developing a processing system for carrying out any herein described method, particularly process 640. Thus, each step of the flow chart may represent a different action performed by a processing system, and may be performed by a respective module of the processing system.

Embodiments make use of a processing system. The processing system can be implemented in numerous ways, with software and/or hardware, to perform the various functions required. A processor is one example of a suitable processing module for a processing system which employs one or more microprocessors that may be programmed using software (e.g., microcode) to perform the required functions. A processing module for a processing system may however be implemented with or without employing a processor, and also may be implemented as a combination of dedicated hardware to perform some functions and a processor (e.g., one or more programmed microprocessors and associated circuitry) to perform other functions.

Examples of processing system components (e.g.,, a processing module) that may be employed in various embodiments of the present disclosure include, but are not limited to, conventional microprocessors, application specific integrated circuits (ASICs), and field-programmable gate arrays (FPGAs).

In various implementations, a processor, processing module or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. An ablation system (200) for performing ablation on the bounds of an anatomical cavity (10, 20) of a subject, the ablation system comprising:
a catheter assembly (205) comprising:
an ablation balloon (210) configured to controllably occlude a portion of the anatomical cavity and be controllable and/or configurable to perform ablation on the bounds of the occluded portion of the anatomical cavity; and
one or more first electrodes (220), located distally to the ablation balloon, wherein each first electrode is configured to obtain a respective electrical response, wherein the electrical response is responsive to a dielectric property of material surrounding the first electrode;
a processing system (250, 500) comprising:
an input interface (251, 510) configured to receive (641) an electrical response from each of the one or more first electrodes of the catheter assembly;
a processing module (252, 520) configured to process (642) any electrical response of the one or more first electrodes received by the input interface to generate an indicator indicating a predicted measure of a dielectric property of liquid upstream of the ablation balloon; and
an optional output interface (253, 530) configured to output (643) the generated indicator,
wherein the one or more first electrodes are spaced apart from the ablation balloon such that, for at least a first time period of at least one second after an ablation procedure is initiated by the ablation balloon, the ablation performed by the ablation balloon has negligible effect on the electrical response obtained by each first electrode.

2. The ablation system of claim 1, wherein the ablation balloon is a cryoablation balloon configured to controllably receive a cooling fluid for performing the ablation on the bounds of the occluded portion of the anatomical cavity.

3. The ablation system of claim 1 or 2, wherein the spacing between the one or more first electrodes and the ablation balloon is at least 2mm.

4. The ablation system of any of claims 1 to 3, wherein the spacing between the one or more first electrodes and the ablation balloon is at least 6mm.

5. The ablation system of any of claims 1 to 4, wherein the position of the one or more first electrodes can be changed independently of the position of the ablation balloon.

6. The ablation system of any of claims 1 to 5, wherein the processing module of the processing system is configured to average the electrical response received by each first electrode to produce the indicator.

7. The ablation system of any of claims 1 to 6, wherein:
the catheter assembly comprises a plurality of catheter electrodes, each catheter electrode being located distally to the ablation balloon and configured to obtain a respective electrical response, wherein the electrical response is responsive to a dielectric property of material surrounding the catheter electrode;
the plurality of catheter electrodes comprises the one or more first electrodes;
the input interface of the processing system is configured to an electrical response from each of the plurality of catheter electrodes of the catheter assembly; and
the processing module is configured to:
process the electrical response of the plurality of catheter electrodes to identify each electrical response of the one or more first electrodes; and
process the identified electrical response of the one or more first electrodes to generate the indicator indicating the predicted measure of the dielectric property of liquid upstream of the ablation balloon.

8. The ablation system of claim 7, wherein the processing module is configured to process the electrical response of the plurality of catheter electrodes to identify each electrical response of the one or more first electrodes by:
receiving, via the input interface, an indication that ablation using the ablation balloon has been initiated; and
identifying each electrical response, of the monitored electrical responses, that has not been significantly distorted or affected by the ablation performed by the ablation balloon during the first time period as an electrical response of the one or more first electrodes.

9. The ablation system of claim 8, wherein the processing module is configured to perform the step of identifying each electrical response, of the monitored electrical responses, that has not been significantly distorted or affected by the ablation performed by the ablation balloon during the first time period by:
identifying any electrical responses that are at or near zero as not being an electrical response of the one or more first electrodes.

10. The ablation system of any of claims 1 to 9, wherein the one or more first electrodes comprises a plurality of first electrodes arranged around a hypothetical circle or spiral.

11. The ablation system of any of claims 1 to 10, wherein the dielectric property of liquid is a dielectric property that changes response to the presence or absence of a dielectric medium in the liquid upstream of the ablation balloon.

12. The ablation system of claim 11, further comprising a dielectric medium injection system configured to inject a dielectric medium upstream of the ablation balloon prior to ablation performed by the ablation balloon.

13. The ablation system of any of claims 1 to 12, wherein the catheter assembly comprises:
a balloon therapy catheter carrying the ablation balloon; and
an electrophysiology catheter carrying the one or more electrodes, wherein the electrophysiology catheter is movable through a lumen of the balloon therapy catheter.

14. A computer-implemented method (640) for aiding ablation performed on a subject, wherein the ablation is performed using a catheter assembly (205) comprising: an ablation balloon (210) configured to controllably occlude a portion of an anatomical cavity (10, 20) and be controllable and/or configurable to perform ablation on the bounds of the occluded portion of the anatomical cavity; and a plurality of catheter electrodes (220), located distally to the ablation balloon, wherein each catheter electrode is configured to obtain a respective electrical response, wherein the electrical response is responsive to a dielectric property of material surrounding the first electrode, the computer-implemented method comprising:
receiving (641), at an input interface, an electrical response from each of the plurality of catheter electrodes of the catheter assembly;
processing, using a processing module, the electrical response of the plurality of catheter electrodes to identify each electrical response of one or more first electrodes of the catheter assembly, the one or more first electrodes being spaced apart from the ablation balloon such that, for at least a first time period of at least one second after an ablation procedure is initiated by the ablation balloon, the ablation performed by the ablation balloon has negligible effect on the electrical response obtained by each first electrode; and
processing (642), using the processing module, the identified electrical response of the one or more first electrodes to generate an indicator indicating the predicted measure of the dielectric property of liquid upstream of the ablation balloon.

15. A computer program product comprising computer program code means which, when executed on a computing device having a processing system, cause the processing system to perform all of the steps of the method according to claim 14.
